# FASCICULE DE BREVET EUROPEEN

(11) **EP 2 488 654 B1**
(45) Date de publication et mention de la délivrance du brevet: **18.01.2017**
(21) Numéro de dépôt: 10763726.6
(22) Date de dépôt: 14.10.2010
(51) Int. Cl.: C12P 7/64, C12P 19/14, C12P 21/00, A23D 9/02, A23J 1/14, C07K 1/14, C11B 1/02, C13K 1/02, C13K 13/00, C13B 20/00

(54) **PROCÉDÉ D'EXTRACTION ENZYMATIQUE EN MILIEU AQUEUX D'HUILES ET DE PROTÉINES A PARTIR DE MATIÈRE VÉGÉTALE**
ENZYMATISCHES VERFAHREN ZUR EXTRAKTION VON ÖLEN UND PROTEINEN AUS EINEM PFLANZENMATERIAL IN EINEM WÄSSRIGEN MEDIUM
METHOD OF ENZYMATIC EXTRACTION OF OILS AND PROTEINS FROM VEGETABLE MATTER IN AQUEOUS MEDIUM

(30) Priorité: 16.10.2009 FR 0957274
(43) Date de publication de la demande: 22.08.2012
(73) Titulaire: Université de Lorraine, 54052 Nancy Cedex (FR)
(72) Inventeur: MUNIGLIA, Lionel, F-54740 Jevoncourt (FR); GIRARDIN, Michel, F-54000 Nancy (FR); PIFFAUT, Bernadette, F-54600 Villiers les Nancy (FR); RICOCHON, Guillaume, F-57770 Moussey (FR)
(74) Mandataire: Icosa
(86) Numéro de dépôt international: PCT/EP2010/065447
(87) Numéro de publication internationale: WO 2011/045387

(56) Documents cités:
- EP-A1- 0 113 165
- EP-B1- 1 658 360
- WO-A1-01/60182
- WO-A1-03/028473
- US-A- 4 478 939
- US-A- 4 904 483
- SENGUPTA R ET AL: "ENZYMATIC EXTRACTION OF MUSTARD SEED AND RICE BRAN", JOURNAL OF THE AMERICAN OIL CHEMISTS' SOCIETY, SPRINGER, BERLIN, DE, vol. 73, no. 6, 1 janvier 1996 (1996-01-01), pages 687-692, XP008039851, ISSN: 0003-021X, DOI: 10.1007/BF02517941
- JUHASZ T ET AL: "Characterization of cellulases and hemicellulases produced by Trichodermareesei on various carbon sources", PROCESS BIOCHEMISTRY, ELSEVIER, NL, vol. 40, no. 11, 1 novembre 2005 (2005-11-01), pages 3519-3525, XP025306694, ISSN: 1359-5113, DOI: 10.1016/J.PROCBIO.2005.03.057 [extrait le 2005-11-01]
- DONAGHY J A ET AL: "Novel screening assay for the detection of phenolic acid esterases", WORLD JOURNAL OF MICROBIOLOGY AND BIOTECHNOLOGY, vol. 10, no. 1, 1994, pages 41-44, XP002588811, ISSN: 0959-3993
- KARLOVIC D J ET AL: "Corn germ oil extraction by a new enzymatic process", ACTA ALIMENTARIA, AKADEMIAI KRADO. BUDAPEST, HU, vol. 23, no. 4, 1 janvier 1994 (1994-01-01), pages 389-400, XP008123782, ISSN: 0139-3006
- SHAO BING ZHANG ET AL: "Optimization of the aqueous enzymatic extraction of rapeseed oil and protein hydrolysates.", JOURNAL OF THE AMERICAN OIL CHEMISTS' SOCIETY, vol. 84, no. 1, 1 janvier 2007 (2007-01-01), pages 97-105, XP002588813, DOI: 10.1007/S11746-006-1004-6
- RAMADAN M F ET AL: "Oil extractability from enzymatically treated goldenberry (Physalis peruviana L.) pomace: range of operational variables.", INTERNATIONAL JOURNAL OF FOOD SCIENCE & TECHNOLOGY, vol. 44, no. 3, 1 mars 2009 (2009-03-01), pages 435-444, XP002588814, DOI: 10.1111/J.1365-2621.2006.01511.X
- PICURIC-JOVANOVIC K ET AL: "Aqueous-enzymatic extraction of plum kernel oil.", FETT/LIPID, vol. 99, no. 12, 1 décembre 1997 (1997-12-01), pages 433-435, XP002588815, DOI: 10.1002/LIPI.19970991205
- H. Domínguez ET AL: "Enzymatic treatment of sunflower kernels before oil extraction", FOOD RESEARCH INTERNATIONAL., vol. 28, no. 6, 1 November 1995 (1995-11-01), pages 537-545, XP055225772, GB ISSN: 0963-9969, DOI: 10.1016/0963-9969(95)00044-5
- "Celluclast 1.5 L, Product Data Sheet", , 7 September 2012 (2012-09-07), XP055225786, Retrieved from the Internet: URL:http://catalog.gusmerenterprises.com/A sset/PDS Celluclast 1.5L 09-07-2012.pdf [retrieved on 2015-11-04]
- "Pectinex Ultra SP-L, Product Data Sheet", , 4 October 2007 (2007-10-04), XP055225789, Retrieved from the Internet: URL:http://catalog.gusmerenterprises.com/A sset/Pectinex Ultra SPL PDS.pdf [retrieved on 2015-11-04]
- KOVACS KRISZTINA ET AL: "Enzymatic hydrolysis of steam-pretreated lignocellulosic materials with Trichoderma atroviride enzymes produced in-house", BIOTECHNOLOGY FOR BIOFUELS, BIOMED CENTRAL LTD, GB, vol. 2, no. 1, 6 July 2009 (2009-07-06), page 14, XP021060600, ISSN: 1754-6834, DOI: 10.1186/1754-6834-2-14
- JENSEN S K ET AL: "Aqueous enzymatic processing of rapeseed for production of high quality products", 1 January 1991 (1991-01-01), CANOLA AND RAPESEED : PRODUCTION, CHEMISTRY, NUTRITION AND PROCESSING TECHNOLOGY, NEW YORK : VAN NOSTRAND REINHOLD, US, PAGE(S) 331 - 344, XP008133562, ISBN: 978-0-442-00295-4

## Description

### Domaine technique

La présente invention concerne un procédé d'extraction enzymatique en milieu aqueux d'huiles, de protéines, et de sucres fermentescibles à partir de matière végétale.

### Etat de la technique

Les procédés traditionnels d'extraction d'huiles font intervenir des solvants organiques, comme l'hexane. L'utilisation de ces solvants entraine de nombreux problèmes de sécurité des installations et des personnels, de santé humaine et de préservation de l'environnement. On recherche en effet à réduire les émissions de composés organiques volatiles (COV).

Depuis une trentaine d'années, plusieurs équipes de recherche dans le monde travaillent à la mise au point de procédés « propres » pour l'extraction de graines d'oléagineux. Ces procédés sont fondés sur une extraction des huiles par voie aqueuse associant enzymes et catalyseurs biologiques. Ces procédés, bien que se voulant écologiques, prévoient d'utiliser malgré tout un solvant ou prévoient une étape de correction du pH par des composés alcalins ou acides. Ces procédés n'ont pas été validés à l'échelle industrielle car les catalyseurs utilisés ne permettaient pas de bons rendements à une telle échelle Le document WO01/60182 décrit un procédé pour séparer les constituants d'une biomasse comprenant une digestion enzymatique en phase aqueuse, dans lequel un mélange d'enzymes pectinases, cellulases et hémicellulases est utilisé.

La présente invention a pour but de proposer un nouveau procédé d'extraction d'huiles à partir d'une matière végétale, exempt des inconvénients énumérés ci-dessus. Plus particulièrement, la présente invention a pour but de proposer un procédé d'extraction des huiles végétales permettant de supprimer l'emploi de tous les solvants organiques et d'obtenir un procédé propre permettant d'extraire des huiles, des protéines et d'autres co-produits de hautes qualités nutritionnelles.

La présente invention a également pour but de proposer un procédé pouvant être mis en oeuvre au niveau industriel avec des rendements intéressants.

La présente invention a également pour but de proposer un procédé permettant d'utiliser tous les produits extraits issus du procédé. Divulgation de l'invention

A cet effet, la présente invention concerne un procédé d'extraction enzymatique en milieu aqueux d'huiles, de protéines et de sucres fermentescibles à partir de matière végétale, comprenant les étapes suivantes :
a) addition d'eau à de la matière végétale présentant une taille de particule appropriée,
b) addition d'un mélange enzymatique contenant au moins une cellulase, au moins une hémicellulase et au moins une pectinase, le rapport entre l'activité de la pectinase et l'activité de la cellulase étant compris entre 0.3 et 2.5, et plus préférentiellement entre 0.35 et 0.45, et le rapport entre l'activité de la pectinase et l'activité de l'hémicellulase étant compris entre 1. 10⁻² et 0.5, et plus préférentiellement entre 1.10⁻² et 2.10⁻², l'activité de la pectinase étant inférieure à 120 µmol/min/ml, et de préférence inférieure à 100 µmol/min/ml, dans lequel la quantité du mélange enzymatique est comprise entre 0.25% et 10% en volume du mélange eau/ matière végétale, de préférence entre 1% et 6%,
c) incubation sous agitation de la matière végétale et du mélange enzymatique pour libérer dans le milieu réactionnel des huiles, des protéines et des sucres fermentescibles, pendant une durée dépendant des rendements recherchés,
d) séparation du milieu réactionnel pour obtenir de l'huile libre, une phase aqueuse contenant des protéines et des sucres fermentescibles, et une phase solide,
e) éventuellement séparation d'une émulsion de l'huile libre ou de la phase aqueuse, et recyclage de l'émulsion dans le milieu réactionnel,
f) séparation des protéines et des sucres fermentescibles de la phase aqueuse.

La présente invention concerne également l'utilisation, dans un procédé tel que défini ci-dessus, d'un mélange enzymatique tel que défini ci-dessus, pour supprimer toute étape de correction du pH dans ledit procédé.

### Mode(s) de réalisation de l'invention

Selon l'invention, le procédé d'extraction enzymatique en milieu aqueux d'huiles, de protéines et de sucres fermentescibles à partir de matière végétale, comprend les étapes suivantes :
a) addition d'eau à de la matière végétale présentant une taille de particule appropriée,
b) addition d'un mélange enzymatique contenant au moins une cellulase, au moins une hémicellulase et au moins une pectinase, le rapport entre l'activité de la pectinase et l'activité de la cellulase étant compris entre 0.3 et 2.5, et le rapport entre l'activité de la pectinase et l'activité de l'hémicellulase étant compris entre 1.10⁻² et 0.5, l'activité de la pectinase étant non nulle et inférieure à 120 µmol/min/ml, dans lequel la quantité du mélange enzymatique est comprise entre 0.25% et 10% en volume du mélange eau/ matière végétale, de préférence entre 1% et 6%,
c) incubation sous agitation de la matière végétale et du mélange enzymatique pour libérer dans le milieu réactionnel des huiles, des protéines et des sucres fermentescibles, pendant une durée dépendant des rendements recherchés,
d) séparation du milieu réactionnel pour obtenir de l'huile libre, une phase aqueuse contenant des protéines et des sucres fermentescibles, et une phase solide,
e) éventuellement séparation d'une émulsion de l'huile libre ou de la phase aqueuse, et recyclage de l'émulsion dans le milieu réactionnel,
f) séparation des protéines et/ou des sucres fermentescibles de la phase aqueuse, en fonction des produits que l'on souhaite récupérer.

En ce qui concerne l'étape a), la taille de particule appropriée de la matière végétale est avantageusement obtenue par broyage de ladite matière végétale. Le broyage doit être le plus fin possible pour favoriser l'action des enzymes. Idéalement, toutes les particules doivent avoir une taille proche de 50 µm, de préférence proche de 10 µm.

Avantageusement, le volume d'eau est minimisé de façon à réduire les effluents à traiter et concentrer les produits extraits. De préférence, la masse d'eau ajoutée à la matière végétale est égale à 1 à 2 fois la masse de ladite matière végétale et ne dépasse pas cette quantité.

De préférence, le procédé selon l'invention comprend en outre, après l'étape a), une étape de désactivation des enzymes endogènes du mélange eau/matière végétale. Cette désactivation se fait de préférence par la chaleur. Le mélange eau/matière végétale est chauffé à une température comprise entre 80°C et 105°C pendant 5 à 20 min. La température est ensuite ramenée à la température utilisée pour l'étape b).

En ce qui concerne l'étape b), le rapport entre l'activité de la pectinase et l'activité de la cellulase est compris entre 0.3 et 2.5, et plus préférentiellement entre 0.35 et 0.45, et le rapport entre l'activité de la pectinase et l'activité de l'hémicellulase est compris entre 1.10⁻² et 0.5, et plus préférentiellement entre 1.10⁻² et 2.10⁻², l'activité de la pectinase étant de préférence inférieure à 100 µmol/min/m.

Plus particulièrement, le mélange enzymatique utilisé contient les activités enzymatiques dans les proportions suivantes :
- cellulases :
   - betaglucosidase : entre 1 µmol/min/ml et 30 µmol/min/ml, de préférence entre 4.5 µmol/min/ml et 13 µmol/min/ml,
   - endocellulase : entre 20 µmol/min/ml et 200 µmol/min/ml, de préférence entre 27 µmol/min/ml et 120 µmol/min/ml,
   - exocellulase : entre 0 et 50 µmol/min/ml, de préférence entre 9 µmol/min/ml et 25.5 µmol/min/ml,
- hemicellulases :
   - arabinanase : entre 300 µmol/min/ml et 2000 µmol/min/ml, de préférence entre 465 µmol/min/ml et 1335 µmol/min/ml,
   - xylanase : entre 0 et 2000 µmol/min/ml, de préférence entre 300 µmol/min/ml et 1700 µmol/min/ml,
   - galactanase : entre 300 µmol/min/ml et 2000 µmol/min/ml, de préférence entre 750 µmol/min/ml et 1000 µmol/min/ml,
- pectinases :
   - endopolygalacturonase : entre 40 µmol/min/ml et 120 µmol/min/ml, de préférence entre 61 µmol/min/ml et 86 µmol/min/ml,
   - pectine méthylestérase : entre 1 µmol/min/ml et 20 µmol/min/ml, de préférence entre 1 µmol/min/ml et 5 µmol/min/ml.

Ces enzymes sont disponibles dans le commerce.

Dans le cas particulier où la matière végétale est le colza, le mélange enzymatique utilisé peut contenir les activités enzymatiques dans les proportions suivantes :
- cellulases :
   - betaglucosidase : entre 3 µmol/min/ml et 6 µmol/min/ml,
   - endocellulase : entre 110 µmol/min/ml et 130 µmol/min/ml,
   - exocellulase : entre 20 µmol/min/ml et 30 µmol/min/ml,
- hemicellulases :
   - arabinanase : entre 1200 µmol/min/ml et 1500 µmol/min/ml,
   - xylanase : entre 1500 µmol/min/ml et 2000 µmol/min/ml,
   - galactanase : entre 800 µmol/min/ml et 1200 µmol/min/ml,
- pectinases :
   - endopolygalacturonase : entre 50 µmol/min/ml et 70 µmol/min/ml,
   - pectine méthylestérase : entre 1 µmol/min/ml et 20 µmol/min/ml.

Dans le cas particulier où la matière végétale est le tournesol, le mélange enzymatique utilisé peut contenir les activités enzymatiques dans les proportions suivantes :
- cellulases :
   - betaglucosidase : entre 11 µmol/min/ml et 14 µmol/min/ml,
   - endocellulase : entre 25 µmol/min/ml et 35 µmol/min/ml,
   - exocellulase : entre 8 µmol/min/ml et 12 µmol/min/ml,
- hemicellulases :
   - arabinanase : entre 450 µmol/min/ml et 550 µmol/min/ml,
   - xylanase : entre 0 et 300 µmol/min/ml,
   - galactanase : entre 700 µmol/min/ml et 900 µmol/min/ml,
- pectinases :
   - endopolygalacturonase : entre 75 µmol/min/ml et 95 µmol/min/ml,
   - pectine méthylestérase : entre 1 µmol/min/ml et 5 µmol/min/ml.

La quantité utilisée du mélange enzymatique tel que défini ci-dessus est comprise entre 0.25% et 10%, de préférence entre 1% et 6%, en volume du mélange eau/matière végétale.

De plus, le mélange enzymatique peut également comprendre une phénolate estérase, de préférence une férulate estérase, dont l'activité est comprise entre 1 µmol/min/ml et 15 µmol/min/ml, de préférence comprise entre 4 µmol/min/ml et 15 µmol/min/ml, et de préférence comprise entre 7.5 µmol/min/ml et 15 µmol/min/ml dans le cas particulier du colza et entre 4 µmol/min/ml et 6 µmol/min/ml dans le cas particulier du tournesol.

Avantageusement, l'incubation selon l'étape c) est réalisée pendant 2 à 20 heures, de préférence entre 4 et 12 heures, à une température comprise entre 25°C et 75°C, de préférence entre 40°C et 60°C, et de préférence autour de 50°C.

D'une manière avantageuse, aucune étape de correction du pH n'est prévue, en particulier lorsque la matière végétale traitée est du colza ou du tournesol.

Le pH du milieu réactionnel doit être compris entre 5.5 et 4.5, et de préférence compris entre 4.8 et 5.

Cependant, il peut être nécessaire dans certains cas de corriger le pH pour correspondre au pH optimum des enzymes. Dans ce cas, on peut utiliser des acides tels que l'acide acétique (E 260) ou l'acide citrique (E 330).

L'agitation prévue à l'étape c) est réalisée de préférence au moyen d'un mélangeur comprenant des pales plates favorisant le mélange et limitant le cisaillement. L'agitation doit être suffisante pour assurer le transfert de chaleur mais minimisée pour éviter l'apparition d'émulsion.

La durée d'incubation est adaptée en fonction de la quantité des produits extraits souhaitée. En effet, du temps d'hydrolyse va dépendre la libération en huiles, en sucres et en protéines. La majorité des protéines se solubilise très rapidement, par exemple en moins d'une heure. Le rendement en huiles se stabilise entre 4 et 6 heures, puis il continue de progresser plus lentement. Les concentrations en sucres fermentescibles augmentent régulièrement jusqu'à 12 heures, et au-delà.

La réaction d'hydrolyse est ensuite stoppée par désactivation des enzymes par chauffage de préférence entre 80°C et 105°C pendant par exemple 5 à 20 minutes.

Puis le milieu réactionnel est séparé, selon l'étape d), en utilisant toutes les techniques de séparation connues adaptées, telle que la centrifugation ou la décantation. Idéalement cette séparation s'effectue sous 3000 g pendant 5 minutes à 80°C.

D'une manière avantageuse, la séparation des produits extraits est réalisée au moyen d'un tricanteur. Un tel appareil est un décanteur centrifuge horizontal pour la séparation continue du milieu réactionnel en trois phases : l'huile libre, une phase aqueuse contenant une partie des protéines et des sucres fermentescibles, et une phase solide constituée par des tourteaux partiellement déshuilés. Le tricanteur permet le recyclage des matières et la gestion de temps d'hydrolyse différents en fonction des produits que l'on souhaite récupérer.

En fonction de la matière végétale dont on veut extraire les huiles, de l'émulsion peut se former dans la phase huileuse ou dans la phase aqueuse.

Le mélange enzymatique utilisé dans l'invention permet de limiter la quantité d'émulsion, en particulier pour le tournesol.

Lorsqu'il se forme de l'émulsion, celle-ci est séparée de la phase avec laquelle elle a été recueillie par exemple au moyen d'un décanteur. L'émulsion peut être recyclée et réinjectée dans le tricanteur afin de subir une nouvelle hydrolyse pour libérer les huiles qu'elle renferme.

Dans le tableau 1 ci-dessous sont indiquées les quantités de produits obtenus à partir de 10 kg de graines (à 50% d'huile) mélangées à 10 kg d'eau, traités selon le procédé de l'invention:

**Tableau 1**

| Produits | Quantité à partir de 10 kg de graines et 10 kg d'eau |
|---|---|
| Huile | 4 à 4.7 kq |
| Phase aqueuse et l'émulsion | 11 à 15 kg |
| Dont sucres | 60 à 100 g/l de phase aqueuse |
| Dont protéines | 25 à 35 g/l de phase aqueuse |
| Tourteaux (le reste de graines) | 2-4 kg |

L'huile libre récupérée est immédiatement stockée sous avec un gaz inerte, tel que l'azote, en attendant son raffinage si nécessaire. Elle contient une grande proportion de tocophérols. Ces antioxydants recherchés sont classiquement entraînés lors de l'étape de désodorisation. Leur présence en grand nombre dans les huiles extraites selon le procédé de l'invention, fait qu'ils sont en partie préservés lors de l'étape de désodorisation.

Les tourteaux sont séchés et stockés ou recyclés pour subir une seconde hydrolyse. Ils peuvent être utilisés pour l'alimentation animale.

Les sucres fermentescibles et les protéines contenus dans la phase aqueuse sont séparés par tout moyen le permettant, notamment par des techniques de filtrations (nano-ultrafiltration), ou par des techniques de précipitation.

Les sucres fermentescibles de la phase aqueuse subissent une fermentation par des micro-organismes (bactéries, levures, champignons) adaptés tels que Saccharomyces cerevisiae, pour produire de l'éthanol, que l'on peut utiliser comme biocarburant.

Les protéines sont extraites de manière importante, les rendements d'extraction étant environ 10 fois supérieurs aux procédés traditionnels d'extraction. Les protéines sont de très bonnes qualités, très peu dénaturées.

Ainsi, tous les produits extraits peuvent être valorisés.

De préférence, le procédé selon l'invention est mis en oeuvre avec de la matière végétale choisie parmi le groupe comprenant les tourteaux gras issus de la première pression et les graines d'oléagineux.

Les oléagineux sont choisis de préférence parmi le groupe comprenant le colza et le tournesol. Le procédé selon l'invention peut aussi être appliqué à des fruits oléagineux, tels que les olives.

Les exemples suivants illustrent la présente invention sans toutefois en limiter la portée.

### Exemples

### Exemples 1 à 5

750 grammes de graines de tournesol sont broyés dans un broyeur à couteau pendant 1min30 (3 fois 30 secondes). Le volume d'eau distillé nécessaire est ajouté au broyat, mélangé et mis à bouillir dans un four à micro-ondes afin de désactiver les enzymes endogènes. Le chauffage est arrêté lorsque l'ébullition commence. Parallèlement, le mélange enzymatique est réalisé dans un bécher. Afin d'éviter un choc thermique, les enzymes étant stockées à 4°C, le bécher est plongé dans un bain-marie à température ambiante. Le bain-marie est alors mis en marche avec une température de consigne de 50°C pour que les enzymes subissent une montée en température graduelle. Les graines broyées sont versées dans un fermenteur de 2L et la régulation de température et l'agitation sont mises en marche. Lorsque le milieu atteint 50°C, le mélange enzymatique est ajouté et la réaction d'hydrolyse débute. Le pH est proche du pH optimum des enzymes et ne nécessite pas de correction. Au bout de 4 heures, le milieu réactionnel est séparé au moyen d'une centrifugeuse à 9000 g pendant 15 min à 20°C. On récupère les différentes phases.

Pour les exemples 1, 3 et 4, l'émulsion séparée de la phase aqueuse n'est pas recyclée.

Pour l'exemple 2, l'émulsion séparée de la phase aqueuse est recyclée.

On mesure le pourcentage massique d'huile contenue dans chaque fraction extraite (huile libre, phase aqueuse + émulsion, phase solide) par rapport à la totalité des huiles recueillies dans les trois fractions (huile libre / phase aqueuse + émulsion / phase solide), sans recyclage de l'émulsion et avec recyclage de l'émulsion.

A titre d'exemple comparatif, un lot de graines est traité de la même manière mais sans enzyme (Exemple 5 comparatif).

Les résultats sont indiqués dans le tableau Il suivant :

**Tableau II**

| | Ex. 1 (inv.) | Ex. 2 (inv.) | Ex. 3 (inv.) | Ex. 4 (inv.) | Ex.5 (comp.) |
|---|---|---|---|---|---|
| Temps (h) | 4 | 4+ recycl. | 12 | 4 | 4 |
| Température (°C) | 50 | 50 | 50 | 50 | 50 |
| Agitation (rpm) | 340 | 340 | 340 | 340 | 450 |
| pH | 5.2 | 5.1 | 4.9 | 5.1 | 5.5 |
| Rapport graines/eau | 0.9 | 0.9 | 0.9 | 0.9 | 0.9 |
| Rapport enzyme/graine | 0.05 | 0.05 | 0.05 | 0.05 | 0 |
| betaglucosidase^{a} | 12.7 | 12.7 | 12.7 | 20.2 | 0 |
| endocellulase^{a} | 27.1 | 27.1 | 27.1 | 10.3 | 0 |
| exocellulase^{a} | 9.4 | 9.4 | 9.4 | 0.7 | 0 |
| arabinanase^{a} | 468.6 | 468.6 | 468.6 | 3.4 | 0 |
| xylanase^{a} | 8.4 | 8.4 | 8.4 | 0 | 0 |
| Galactanase^{a} | 775.8 | 775.8 | 775.8 | 153 | 0 |
| endopolygalacturonase^{a} | 85.1 | 85.1 | 85.1 | 76.9 | 0 |
| pectine méthylestérase^{a} | 1.8 | 1.8 | 1.8 | 1.7 | 0 |
| Férulate estérase^{a} | 3.8 | 3.8 | 3.8 | 4.2 | 0 |
| Masse d'huile libre (g) | 270.3 | 335.7 | 329.4 | 278.7 | 127.9 |
| Masse huile émulsion (g) | 26.3 | 14.6 | 33.3 | 56.3 | 26.7 |
| Masse huile phase solide (g) | 56.5 | 32.18 | 35.6 | 72.7 | 197.0 |
| Masse totale huile (g) | 353.1 | 383.2 | 398.3 | 407.8 | 351.6 |
| Masse huile libre (%) | 76.5 | 87.6 | 82.7 | 68.5 | 36.4 |
| Masse huile émulsion (%) | 7.5 | 3.8 | 8.4 | 13.2 | 7.6 |
| Masse huile phase solide (%) | 16.0 | 8.4 | 9.0 | 17.3 | 56.0 |

| | | | | | |
|---|---|---|---|---|---|
| ^{a} activité en µmol/min/ml | | | | | |

Le procédé selon l'invention permet d'obtenir des résultats proches de ceux obtenus par les industries traditionnelles utilisant des solvants. L'émulsion obtenue contient très peu d'huile. A titre comparatif, il reste généralement 4-5% d'huile dans l'émulsion dans les procédés traditionnels avec solvant, et il reste 10-15% d'huile dans l'émulsion dans les procédés connus sans solvant, selon les graines.

Les concentrations en sucres varient de 40 à 90 g/l de phase aqueuse, pour un temps d'hydrolyse de 4 à 12 heures.

### Exemples 6 à 12

Les Exemples 6 à 12 sont traités de la même manière que les exemples ci-dessus mais sur des graines de colza et sans recyclage de l'émulsion séparée de la phase aqueuse.

Pour les exemples 6 et 7, la durée d'hydrolyse est de 15 heures. L'exemple 6 est exempt d'activité férulate estérase.

A titre d'exemple comparatif, un lot de graines est traité de la même manière mais sans enzyme (Exemple 10 comparatif) ou avec les mêmes enzymes mais avec des rapports entre activités ne faisant pas partie de l'invention.

Les résultats sont indiqués dans le tableau III suivant :

**Tableau III**

| | Ex. 6 (inv.) | Ex. 7 (inv.) | Ex. 8 (inv.) | Ex. 9 (inv.) | Ex. 10 (comp.) | Ex. 11 (comp.) | Ex. 12 (comp.) |
|---|---|---|---|---|---|---|---|
| Temps (h) | 15 | 15 | 4 | 4 | 4 | 4 | 4 |
| Température (°C) | 50 | 50 | 50 | 50 | 50 | 50 | 50 |
| Agitation | 300 | 300 | 300 | 300 | 450 | 300 | 330 |
| pH | 5.0 | 4.9 | 5.0 | 4.9 | 5.2 | 5.0 | 5.1 |
| Rapport graines/eau | 0.67 | 0.67 | 0.67 | 0.67 | 0.67 | 0.67 | 0.67 |
| Rapport enzyme/graine | 0.05 | 0.05 | 0.05 | 0.05 | 0 | 0.05 | 0.05 |
| betaglucosidase^{a} | 8 | 8 | 8 | 4.7 | 0 | 1.39 | 24.3 |
| endocellulase^{a} | 14 | 14 | 14 | 119.8 | 0 | 218.2 | 35.8 |
| exocellulase^{a} | 2 | 2 | 2 | 25.3 | 0 | 29.2 | 13.0 |
| arabinanase^{a} | 10 | 10 | 10 | 1334.8 | 0 | 1511.1 | 697.0 |
| xylanase^{a} | 0 | 0 | 0 | 1713.9 | 0 | 4139.1 | 12.6 |
| Galactanase^{a} | 450 | 450 | 450 | 1000.8 | 0 | 175.2 | 907.2 |
| Endopolygalacturonase^{a} | 57.9 | 57.9 | 57.9 | 61.7 | 0 | 0 | 126.62 |
| pectine méthylestérase^{a} | 3.5 | 3.5 | 3.5 | 0.3 | 0 | 0 | 0.95 |
| Férulate estérase^{a} | 0 | 27 | 27 | 6.5 | 0 | 6.5 | 3.8 |
| Masse d'huile libre (g) | 180.9 | 202.0 | 51.7 | 118.8 | 15.0 | 91.4 | 70.0 |
| Masse huile émulsion (g) | 58.5 | 45.7 | 176.53 | 114.0 | 188.8 | 114.5 | 158.9 |
| Masse huile phase solide (g) | 44.3 | 39.4 | 66.42 | 63.67 | 95.9 | 98.4 | 101.1 |
| Masse totale huile(g) | 284 | 287.3 | 295.2 | 296.6 | 299.7 | 304.6 | 330.1 |
| Masse huile libre (%) | 63.7 | 70.3 | 17.5 | 40.1 | 5 | 30 | 21.2 |
| Masse huile émulsion (%) | 20.6 | 15.9 | 59.8 | 38.4 | 63 | 37.6 | 48.1 |
| Masse huile phase solide (%) | 15.6 | 13.7 | 22.5 | 21.5 | 32 | 32.3 | 30.6 |

| | | | | | | | |
|---|---|---|---|---|---|---|---|
| ^{a} activité en µmol/min/ml | | | | | | | |

Les exemples 6 à 9 mettent en évidence le rôle de la férulate estérase.

L'exemple 9 d'une durée de 4 heures, montre le rôle du temps d'hydrolyse. Tout comme les exemples 7 et 8 réalisés avec le même mélange enzymatique mais avec une durée d'hydrolyse différente.

Les concentrations en sucres varient de 40 à 90 g/l de phase aqueuse, pour un temps d'hydrolyse de 4 à 12 heures.

Les concentrations en protéines sont de 30 à 35 g/l de phase aqueuse dès la première heure d'hydrolyse.

Les exemples comparatifs 10 à 12 montrent les avantages des rapports d'activité des mélanges enzymatiques utilisés dans le procédé de l'invention par rapport à des procédés sans enzyme ou utilisant des mélanges enzymatiques présentant des rapports d'activité différents de ceux de la présente invention. En particulier, le procédé utilisant les mélanges enzymatiques selon l'invention permet d'obtenir une plus grande masse d'huile libre que les procédés utilisant des mélanges enzymatiques présentant des rapports d'activité différents de ceux de la présente invention.

## Revendications

1. Procédé d'extraction enzymatique en milieu aqueux d'huiles, de protéines et de sucres fermentescibles à partir de matière végétale, comprenant les étapes suivantes :
a) addition d'eau à de la matière végétale présentant une taille de particule appropriée,
b) addition d'un mélange enzymatique contenant au moins une cellulase, au moins une hémicellulase et au moins une pectinase, le rapport entre l'activité de la pectinase et l'activité de la cellulase étant compris entre 0.3 et 2.5, et plus préférentiellement entre 0.35 et 0.45, et le rapport entre l'activité de la pectinase et l'activité de l'hémicellulase étant compris entre 1.10⁻² et 0.5, et plus préférentiellement entre 1.10⁻² et 2.10⁻², l'activité de la pectinase étant inférieure à 120 µmol/min/ml, et de préférence inférieure à 100 µmol/min/ml, dans lequel la quantité du mélange enzymatique est comprise entre 0.25% et 10% en volume du mélange eau/matière végétale, de préférence entre 1% et 6%,
c) incubation sous agitation de la matière végétale et du mélange enzymatique pour libérer dans le milieu réactionnel des huiles, des protéines et des sucres fermentescibles, pendant une durée dépendant des rendements recherchés,
d) séparation du milieu réactionnel pour obtenir de l'huile libre, une phase aqueuse contenant des protéines et des sucres fermentescibles, et une phase solide,
e) éventuellement séparation d'une émulsion de l'huile libre ou de la phase aqueuse, et recyclage de l'émulsion dans le milieu réactionnel,
f) séparation des protéines et des sucres fermentescibles de la phase aqueuse.

2. Procédé selon la revendication **1,** dans lequel la taille de particule appropriée de la matière végétale est obtenue par broyage de ladite matière végétale.

3. Procédé selon l'une quelconque des revendications **1** et **2,** dans lequel la masse d'eau ajoutée à la matière végétale est égale à 1 à 2 fois la masse de ladite matière végétale.

4. Procédé selon l'une quelconque des revendications précédentes, comprenant en outre, après l'étape a), une étape de désactivation des enzymes endogènes du mélange eau/matière végétale.

5. Procédé selon l'une quelconque des revendications précédentes, dans lequel le mélange enzymatique contient les activités enzymatiques dans les proportions suivantes :
- cellulases :
- betaglucosidase : entre 1 µmol/min/ml et 30 µmol/min/ml, de préférence entre 4.5 µmol/min/ml et 13 µmol/min/ml,
- endocellulase : entre 20 µmol/min/ml et 200 µmol/min/ml, de préférence entre 27 µmol/min/ml et 120 µmol/min/ml,
- exocellulase : entre 0 et 50 µmol/min/ml, de préférence entre 9 µmol/min/ml et 25.5 µmol/min/ml.
- hémicellulases :
- arabinanase : entre 300 µmol/min/ml et 2000 µmol/min/ml, de préférence entre 465 µmol/min/ml et 1335 µmol/min/ml,
- xylanase : entre 0 et 2000 µmol/min/ml, de préférence entre 300 µmol/min/ml et 1700 µmol/min/ml,
- galactanase : entre 300 µmol/min/ml et 2000 µmol/min/ml, de préférence entre 750 µmol/min/ml et 1000 µmol/min/ml.
- pectinases :
- endopolygalacturonase : entre 40 µmol/min/ml et 120 µmol/min/ml, de préférence entre 61 µmol/min/ml et 86 µmol/min/ml,
- pectine méthylestérase : entre 1 µmol/min/ml et 20 µmol/min/ml, de préférence entre 1 µmol/min/ml et 5 µmol/min/ml.

6. Procédé selon l'une quelconque des revendications précédentes, dans lequel le mélange enzymatique comprend en outre une phénolate estérase, de préférence une férulate estérase, dont l'activité est comprise entre 1 µmol/min/ml et 15 µmol/min/ml, et de préférence entre 4 µmol/min/ml et 15 µmol/min/ml.

7. Procédé selon l'une quelconque des revendications précédentes, dans lequel l'incubation selon l'étape c) est réalisée pendant 2 à 20 heures, à une température comprise entre 25°C et 75°C.

8. Procédé selon l'une quelconque des revendications précédentes, dans lequel l'agitation prévue à l'étape c) est réalisée au moyen d'un mélangeur comprenant des pales plates favorisant le mélange et limitant le cisaillement.

9. Procédé selon l'une quelconque des revendications précédentes, dans lequel aucune étape de correction du pH n'est prévue.

10. Procédé selon l'une quelconque des revendications précédentes, dans lequel l'étape de séparation d) est réalisée au moyen d'un tricanteur.

11. Procédé selon l'une quelconque des revendications précédentes, dans lequel la matière végétale est choisie parmi le groupe comprenant les tourteaux gras issus de la première pression et les graines d'oléagineux.

12. Procédé selon la revendication 11, dans lequel les oléagineux sont choisis parmi le groupe comprenant le colza et le tournesol.

13. Utilisation, dans un procédé selon l'une quelconque des revendications précédentes, d'un mélange enzymatique contenant au moins une cellulase, au moins une hémicellulase et au moins une pectinase, le rapport entre l'activité de la pectinase et l'activité de la cellulase étant compris entre 0.3 et 2.5, et plus préférentiellement entre 0.35 et 0.45, et le rapport entre l'activité de la pectinase et l'activité de l'hémicellulase étant compris entre 1.10⁻² et 0.5, et plus préférentiellement entre 1.10⁻² et 2.10⁻², l'activité de la pectinase étant inférieure à 120 µmol/min/ml, et de préférence inférieure à 100 µmol/min/ml, dans lequel la quantité du mélange enzymatique est comprise entre 0.25% et 10% en volume du mélange eau/matière végétale, de préférence entre 1% et 6%, pour supprimer toute étape de correction du pH dans ledit procédé.

14. Utilisation selon la revendication **13,** selon laquelle le mélange enzymatique contient les activités enzymatiques dans les proportions suivantes :
- cellulases :
- betaglucosidase : entre 1 µmol/min/ml et 30 µmol/min/ml, de préférence entre 4.5 µmol/min/ml et 13 µmol/min/ml,
- endocellulase : entre 20 µmol/min/ml et 200 µmol/min/ml, de préférence entre 27 µmol/min/ml et 120 µmol/min/ml,
- exocellulase : entre 0 et 50 µmol/min/ml, de préférence entre 9 µmol/min/ml et 25.5 µmol/min/ml.
- hémicellulases :
- arabinanase : entre 300 µmol/min/ml et 2000 µmol/min/ml, de préférence entre 465 µmol/min/ml et 1335 µmol/min/ml,
- xylanase : entre 0 et 2000 µmol/min/ml, de préférence entre 300 µmol/min/ml et 1700 µmol/min/ml,
- galactanase : entre 300 µmol/min/ml et 2000 µmol/min/ml, de préférence entre 750 µmol/min/ml et 1000 µmol/min/ml.
- pectinases :
- endopolygalacturonase : entre 40 µmol/min/ml et 120 µmol/min/ml, de préférence entre 61 µmol/min/ml et 86 µmol/min/ml,
- pectine méthylestérase : entre 1 µmol/min/ml et 20 µmol/min/ml, de préférence entre 1 µmol/min/ml et 5 µmol/min/ml.

## Patentansprüche

1. Verfahren zur enzymatischen Extraktion in wässrigem Medium von Ölen, Proteinen und fermentierbaren Zuckern aus pflanzlichem Material, umfassend die folgenden Schritte:
a) Zugeben von Wasser zu pflanzlichem Material, das eine geeignete Partikelgröße aufweist,
b) Zugeben einer enzymatischen Mischung, enthaltend mindestens eine Cellulase, mindestens eine Hemicellulase und mindestens eine Pectinase, wobei das Verhältnis zwischen der Aktivität der Pectinase und der Aktivität der Cellulase im Bereich zwischen 0,3 und 2,5 und bevorzugt zwischen 0,35 und 0,45 liegt und das Verhältnis zwischen der Aktivität der Pectinase und der Aktivität der Hemicellulase im Bereich zwischen 1,10⁻² und 0,5, und bevorzugt zwischen 1,10⁻² und 2,10⁻² liegt, wobei die Aktivität der Pectinase kleiner als 120 µmol/min/ml und vorzugsweise kleiner als 100 µmol/min/ml ist, wobei die Menge der enzymatischen Mischung im Bereich zwischen 0,25 Vol% und 10 Vol% der Mischung Wasser/pflanzliches Material, vorzugsweise zwischen 1 % und 6 % liegt,
c) Inkubieren unter Rühren des pflanzlichen Materials und der enzymatischen Mischung, um im Reaktionsmedium Öle, Proteine und fermentierbare Zucker während einer Dauer freizusetzen, die von den gesuchten Wirkungen abhängt,
d) Trennen des Reaktionsmediums, um freies Öl, eine wässrige Phase, die Proteine und fermentierbare Zucker umfasst, und eine feste Phase zu erhalten,
e) eventuell Trennen einer Emulsion des freien Öls oder der wässrigen Phase und Recyceln der Emulsion im Reaktionsmedium,
f) Trennen der Proteine und der fermentierbaren Zucker der wässrigen Phase.

2. Verfahren nach Anspruch **1,** wobei die geeignete Partikelgröße des pflanzlichen Materials durch Mahlen des pflanzlichen Materials erhalten wird.

3. Verfahren nach einem der Ansprüche **1** und **2,** wobei die Masse von Wasser, das dem pflanzlichen Material zugegeben wird, gleich 1 bis 2 Mal die Masse des pflanzlichen Materials ist.

4. Verfahren nach einem der vorhergehenden Ansprüche, außerdem umfassend, nach Schritt a), einen Schritt des Deaktivierens der endogenen Enzyme der Mischung Wasser/pflanzliches Material.

5. Verfahren nach einem der vorhergehenden Ansprüche, wobei die enzymatische Mischung die enzymatischen Aktivitäten in den folgenden Proportionen enthält:
- Cellulasen:
- Betaglucosidase: zwischen 1 µmol/min/ml und 30 µmol/min/ml, vorzugsweise zwischen 4,5 µmol/min/ml und 13 µmol/min/ml,
- Endocellulase: zwischen 20 µmol/min/ml und 200 µmol/min/ml, vorzugsweise zwischen 27 µmol/min/ml und 120 µmol/min/ml,
- Exocellulase: zwischen 0 und 50 µmol/min/ml, vorzugsweise zwischen 9 µmol/min/ml und 25,5 µmol/min/ml.
- Hemicellulasen:
- Arabinanase: zwischen 300 µmol/min/ml und 2000 µmol/min/ml, vorzugsweise zwischen 465 µmol/min/ml und 1335 µmol/min/ml,
- Xylanase: zwischen 0 und 2000 µmol/min/ml, vorzugsweise zwischen 300 µmol/min/ml und 1700 µmol/min/ml,
- Galactanase: zwischen 300 µmol/min/ml und 2000 µmol/min/ml, vorzugsweise zwischen 750 µmol/min/ml und 1000 µmol/min/ml.
- Pectinasen:
- Endopolygalacturonase: zwischen 40 µmol/min/ml und 120 µmol/min/ml, vorzugsweise zwischen 61 µmol/min/ml und 86 µmol/min/ml,
- Pectinmethylesterase: zwischen 1 µmol/min/ml und 20 µmol/min/ml, vorzugsweise zwischen 1 µmol/min/ml und 5 µmol/min/ml.

6. Verfahren nach einem der vorhergehenden Ansprüche, wobei die enzymatische Mischung außerdem eine Phenolatesterase umfasst, vorzugsweise eine Ferulatesterase, deren Aktivität zwischen 1 µmol/min/ml und 15 µmol/min/ml und vorzugsweise zwischen 4 µmol/min/ml und 15 µmol/min/ml liegt.

7. Verfahren nach einem der vorhergehenden Ansprüche, wobei die Inkubation gemäß Schritt c) während 2 bis 20 Stunden bei einer Temperatur zwischen 25 °C und 75 °C durchgeführt wird.

8. Verfahren nach einem der vorhergehenden Ansprüche, wobei das Rühren, vorgesehen in Schritt c), mit Hilfe eines Mischers, umfassend flache Schaufeln, die die Mischung begünstigen und die Scherung verhindern, durchgeführt wird.

9. Verfahren nach einem der vorhergehenden Ansprüche, wobei kein Schritt des Korrigierens des pH-Werts vorgesehen ist.

10. Verfahren nach einem der vorhergehenden Ansprüche, wobei der Schritt des Trennens d) mit Hilfe eines Tricanters durchgeführt wird.

11. Verfahren nach einem der vorhergehenden Ansprüche, wobei das pflanzliche Material ausgewählt ist aus der Gruppe, umfassend die fetten Presskuchen aus der ersten Pressung und die Ölsaaten.

12. Verfahren nach Anspruch **11,** wobei die Ölsaaten ausgewählt sind aus der Gruppe, umfassend Raps und Sonnenblumen.

13. Verwendung, in einem Verfahren nach einem der vorhergehenden Ansprüche, einer enzymatischen Mischung, enthaltend mindestens eine Cellulase, mindestens eine Hemicellulase und mindestens eine Pectinase, wobei das Verhältnis zwischen der Aktivität der Pectinase und der Aktivität der Hemicellulase im Bereich zwischen 0,3 und 2,5 und bevorzugt zwischen 0,35 und 0,45 liegt und das Verhältnis zwischen der Aktivität der Pectinase und der Aktivität der Hemicellulase im Bereich zwischen 1,10⁻² und 0,5 und bevorzugt zwischen 1,10⁻² und 2,10⁻² liegt, wobei die Aktivität der Pectinase kleiner als 120 µmol/min/ml und vorzugsweise kleiner als 100 µmol/min/ml ist, wobei die Menge der enzymatischen Mischung im Bereich zwischen 0,25 Vol% und 10 Vol% der Mischung Wasser/pflanzliches Material, vorzugsweise zwischen 1 % und 6 % liegt, um jeden Schritt des Korrigierens des pH-Werts in dem Verfahren zu unterdrücken.

14. Verwendung nach Anspruch **13,** wobei die enzymatische Mischung die enzymatischen Aktivitäten in den folgenden Proportionen enthält:
- Cellulasen:
- Betaglucosidase: zwischen 1 µmol/min/ml und 30 µmol/min/ml, vorzugsweise zwischen 4,5 µmol/min/ml und 13 µmol/min/ml,
- Endocellulase: zwischen 20 µmol/min/ml und 200 µmol/min/ml, vorzugsweise zwischen 27 µmol/min/ml und 120 µmol/min/ml,
- Exocellulase: zwischen 0 und 50 µmol/min/ml, vorzugsweise zwischen 9 µmol/min/ml und 25,5 µmol/min/ml.
- Hemicellulasen:
- Arabinanase: zwischen 300 µmol/min/ml und 2000 µmol/min/ml, vorzugsweise zwischen 465 µmol/min/ml und 1335 µmol/min/ml,
- Xylanase: zwischen 0 und 2000 µmol/min/ml, vorzugsweise zwischen 300 µmol/min/ml und 1700 µmol/min/ml,
- Galactanase: zwischen 300 µmol/min/ml und 2000 µmol/min/ml, vorzugsweise zwischen 750 µmol/min/ml und 1000 µmol/min/ml.
- Pectinasen:
- Endopolygalacturonase: zwischen 40 µmol/min/ml und 120 µmol/min/ml, vorzugsweise zwischen 61 µmol/min/ml und 86 µmol/min/ml,
- Pectinmethylesterase: zwischen 1 µmol/min/ml und 20 µmol/min/ml, vorzugsweise zwischen 1 µmol/min/ml und 5 µmol/min/ml.

## Claims

1. Method of enzymatic extraction of oils, proteins and fermentable sugars from vegetable material in aqueous medium, comprising the following steps:
a) adding water to a vegetable material having a suitable particle size,
b) adding an enzyme mixture containing at least one cellulase, at least one hemicellulase and at least one pectinase, the ratio between the pectinase activity and the cellulase activity being comprised between 0.3 and 2.5, and more preferably between 0.35 and 0.45, and the ratio between the pectinase activity and the hemicellulase activity being comprised between 1.10⁻² and 0.5, and more preferably between 1.10⁻² and 2.10⁻², the pectinase activity being less than 120 µmol/min/ml, and preferably less than 100 µmol/min/ml, wherein the amount of the enzyme mixture is comprised between 0.25% and 10% by volume of the water/vegetable material mixture, preferably between 1% and 6%,
c) incubating with stirring the vegetable material and the enzyme mixture in order to release in the reaction medium oils, proteins and fermentable sugars, for a duration depending on the sought yields,
d) separating the reaction medium in order to obtain free oil, an aqueous phase containing proteins and fermentable sugars, and a solid phase,
e) optionally separating an emulsion of the free oil or of the aqueous phase, and recycling the emulsion in the reaction medium,
f) separating the proteins and fermentable sugars from the aqueous phase.

2. Method according to claim **1,** wherein the suitable particle size of the vegetable material is obtained by milling said vegetable material.

3. Method according to any one of claims **1** and **2,** wherein the mass of water added to the vegetable material is equal to 1 to 2 times the mass of said vegetable material.

4. Method according to any one of the previous claims, further comprising, after step a), a step of deactivation of the endogenous enzymes of the water/vegetable material mixture.

5. Method according to any one of the previous claims, wherein the enzyme mixture contains the enzyme activities in the following proportions:
- cellulases:
- betaglucosidase: between 1 µmol/min/ml and 30 µmol/min/ml, preferably between 4.5 µmol/min/ml and 13 µmol/min/ml,
- endocellulase: between 20 µmol/min/ml and 200 µmol/min/ml, preferably between 27 µmol/min/ml and 120 µmol/min/ml,
- exocellulase: between 0 and 50 µmol/min/ml, preferably between 9 µmol/min/ml and 25.5 µmol/min/ml.
- hemicellulases:
- arabinanase: between 300 µmol/min/ml and 2000 µmol/min/ml, preferably between 465 µmol/min/ml and 1335 µmol/min/ml,
- xylanase: between 0 and 2000 µmol/min/ml, preferably between 300 µmol/min/ml and 1700 µmol/min/ml,
- galactanase: between 300 µmol/min/ml and 2000 µmol/min/ml, preferably between 750 µmol/min/ml and 1000 µmol/min/ml.
- pectinases:
- endopolygalacturonase: between 40 µmol/min/ml and 120 µmol/min/ml, preferably between 61 µmol/min/ml and 86 µmol/min/ml,
- pectin methylesterase: between 1 µmol/min/ml and 20 µmol/min/ml, preferably between 1 µmol/min/ml and 5 µmol/min/ml.

6. Method according to any one of the previous claims, wherein the enzyme mixture further comprises a phenolate esterase, preferably a ferulate esterase, whose activity is between 1 µmol/min/ml and 15 µmol/min/ml, and preferably between 4 µmol/min/ml and 15 µmol/min/ml.

7. Method according to any one of the previous claims, wherein the incubation according to step c) is performed between 2 to 20 hours, at a temperature between 25°C and 75°C.

8. Method according to any one of the previous claims, wherein the stirring provided in step c) is achieved by means of a mixer comprising flat blades promoting mixing and limiting shear.

9. Method according to any one of the previous claims, wherein no step for correcting the pH is planned.

10. Method according to any one of the previous claims, wherein the separation step d) is achieved by means of a tricanter.

11. Method according to any one of the previous claims, wherein the vegetable material is selected from the group comprising fat cakes from the first pressing and oilseeds.

12. Method according to claim 11, wherein the oilseeds are selected from the group comprising rapeseed and sunflower.

13. Use in a method according to any one of the previous claims of an enzyme mixture containing at least one cellulase, at least one hemicellulase and at least one pectinase, the ratio between the pectinase activity and the cellulase activity being between 0.3 and 2.5, and more preferably between 0.35 and 0.45, and the ratio between the pectinase activity and the hemicellulase activity being between 1.10⁻² and 0.5, and more preferably between 1.10⁻² and 2.10⁻², the pectinase activity being less than 120 µmol/min/ml, and preferably less than 100 µmol/min/ml, wherein the amount of the enzyme mixture is between 0.25% and 10% by volume of the water/vegetable material mixture, preferably between 1% and 6%, to suppress any pH correction step in said method.

14. Use according to claim **13,** wherein the enzyme mixture contains the enzyme activities in the following proportions:
- cellulases:
- betaglucosidase: between 1 µmol/min/ml and 30 µmol/min/ml, preferably between 4.5 µmol/min/ml and 13 µmol/min/ml,
- endocellulase: between 20 µmol/min/ml and 200 µmol/min/ml, preferably between 27 µmol/min/ml and 120 µmol/min/ml,
- exocellulase: between 0 and 50 µmol/min/ml, preferably between 9 µmol/min/ml and 25.5 µmol/min/ml.
- hemicellulases:
- arabinanase: between 300 µmol/min/ml and 2000 µmol/min/ml, preferably between 465 µmol/min/ml and 1335 µmol/min/ml,
- xylanase: between 0 and 2000 µmol/min/ml, preferably between 300 µmol/min/ml and 1700 µmol/min/ml,
- galactanase: between 300 µmol/min/ml and 2000 µmol/min/ml, preferably between 750 µmol/min/ml and 1000 µmol/min/ml.
- pectinases:
- endopolygalacturonase: between 40 µmol/min/ml and 120 µmol/min/ml, preferably between 61 µmol/min/ml and 86 µmol/min/ml,
- pectin methylesterase: between 1 µmol/min/ml and 20 µmol/min/ml, preferably between 1 µmol/min/ml and 5 µmol/min/ml.
